Europäisches Patentamt

European Patent Office

Office européen des brevets

(11) Publication number: **0 212 546 B1**

# EUROPEAN PATENT SPECIFICATION

(45) Date of publication of patent specification: **01.04.92**    (51) Int. Cl.⁵: **C07H 21/00**, //C12Q1/68

(21) Application number: **86111137.5**

(22) Date of filing: **12.08.86**

(54) **Method for labeling polynucleotide sequences.**

(30) Priority: **13.08.85 US 765288**

(43) Date of publication of application:
**04.03.87 Bulletin 87/10**

(45) Publication of the grant of the patent:
**01.04.92 Bulletin 92/14**

(84) Designated Contracting States:
**AT BE CH DE FR GB IT LI LU NL SE**

(56) References cited:
**EP-A- 0 097 373**
**EP-A- 0 117 440**
**EP-A- 0 156 287**

**BIOCHEMICA ET BIOPHYSICA ACTA, vol. 91, 1964, pages 59-66; E. KRIEK et al.: "Methylation of deoxyribonucleic acid by diazomethane"**

(73) Proprietor: **ENZO BIOCHEM, INC.**
**60 Executive Boulevard**
**Farmingdale, New York 11735(US)**

(72) Inventor: **Stavrianopoulos, Jannis**
**1380 Riverside Drive Apartment 8E**
**New York New York 10033(US)**
Inventor: **Rabbani, Elazar**
**69 Fifth Avenue**
**New York New York 10003(US)**

(74) Representative: **Vossius & Partner**
**Siebertstrasse 4 P.O. Box 86 07 67**
**W-8000 München 86(DE)**

EP 0 212 546 B1

**Description**

Recent advances in molecular biology have allowed the development of improved methods for detecting infectious agents in clinical specimens. One promising method is that of hybridizing a polynucleotide probe to a polynucleotide analyte of an infectious agent. The hybridization can be carried out in intact cells or in sample extracts. This technique has the advantage over immunoassays, e.g. ELISA, in that the detection of the infectious agent can be achieved even when epitopes of the particular infectious agent are not being expressed. This permits the detection of an infectious agent that might otherwise escape detection.

Polynucleotide probes consist essentially of two components: a polynucleotide sequence and a signalling moiety. The polynucleotide sequence is capable of hybridizing to a segment of the target genome. The sequence can be comprised of deoxyribonucleotides or ribonucleotides and can range in length from about fifteen to several thousand nucleotides. The signalling moiety is a fragment or molecule that provides a signal or the means for generating a signal to indicate that hybridization has taken place.

First generation signalling moieties comprised $^{32}$P atoms and provided a radioactive signal. The use of these signalling moieties has drawbacks in that $^{32}$P has a 14-day half-life, poses health risks, and requires special disposal facilities. This led to the development of the current second-generation non-isotopic signalling moieties that permit one to generate a signal by means of fluorescence, luminescence or a chromogen. These second generation probes have a drawback in that the signalling moiety is attached to the base portions of the polynucleotide; the signalling moiety can thus sterically interfere with proper base pairing.

Recently, a polynucleotide probe was modified in which a fluorescent signalling moiety was attached to the sugar portions of the polynucleotide. The method comprised depurinating the polynucleotide at a high temperature and acidic pH to generate sugar aldehydes. Acriflavin dyes were attached to the sugars via a Schiff base at the 1 position of the sugar. See "Fluorescent Labeling of DNA by Covalent Binding of Acriflavin", J.W. Levinson, A. deSostoa, L.F. Kiebes, and J.J. McCormick, Biophysical Journal, 16: 91a (1976). This method has a drawback, in that the acidic conditions required for depurination catalyzes the formation of cross-links between the sugar aldehydes and amino bases. The cross-links interfere with proper base hybridization to the target genome. A second drawback is that one dye per two hundred sugars was the highest ratio that could be achieved that would still permit the DNA to hybridize to itself at 85% of the value of the control DNA.

The ability to selectively remove pyrimidines and N-quaternized purines from polynucleotides is known. However, the removal in the past has not been with the intent of labeling a polynucleotide sequence.

Rather, it was to break the polynucleotide chain to determine its base sequence. See Allan M. Maxam and Walter Gilbert, Methods In Enzymology, 65, 449-560, 1980.

It is an object of this invention to provide a method for labeling a polynucleotide sequence by attaching a signalling moiety to the sugar portion of the polynucleotide sequence.

It is another object of this invention to provide a method for generating many sugar aldehydes under conditions wherein cross-linking between the aldehyde and amino bases is severely minimized.

This invention relates to a method for labeling a polynucleotide sequence comprising generating a sugar aldehyde on that sequence and then attaching a signalling moiety to the sequence by means of the sugar aldehyde. The sugar aldehyde may be generated by removing an N-alkylated adenine or guanine base from a modified polynucleotide sequence, or a uracil base from a polynucleotide sequence. Removal of the N-alkylated adenine or guanine base (depurination) is carried out by hydrolysis which generates the sugar aldehyde. Removal of the uracil base is carried out by reacting the polynucleotide sequence with hydroxylamine. The hydroxylamine cleaves the uracil base while forming a sugar oxime. Contacting the sugar oxime with a ketone-containing compound generates the sugar aldehyde.

The depurination is carried out from about pH 5.0 to about pH 7.5, preferably from about pH 6.0 to about 7.5 at a temperature from about 40° to about 60°C. A signalling moiety or bridging moiety is present during the depurination step.

A method is provided for modifying a polynucleotide sequence for use as a probe. The signalling moiety is attached to the sugar portion of the sequence. The method comprises removing a preselected purine or uracil base from the sequence to generate apurinic or apyrimidinic sites. The sugars at those sites contain either an aldehyde, i.e. a sugar aldehyde, or a Schiff base at the 1-position depending upon whether the base removed is a quaternized purine or uracil, respectively. Sugars containing a Schiff base as a result of uracil cleavage are hydrolyzed to the aldehyde, i.e. a sugar aldehyde is formed. A signalling moiety is then attached to the polynucleotide sequence via the sugar aldehyde.

The preselected purine bases adaptable to this invention must contain at least one quaternized nitrogen; i.e. N-1, N-3, and N-7 position. Quaternizing the nitrogen destabilizes the glycosidic bond permitting the cleavage of the purine from the polynucleotide at neutral conditions. The carrying out of this reaction at neutral conditions minimizes the formation of cross-links between the aldehyde group of the sugar and the amino bases. Release of the purine generates a sugar aldehyde at the site of cleavage.

Purine nucleotide as used in this invention means either an individual nucleotide or a nucleotide that is integral to a polynucleotide. Quaternization of a purine nucleotide is generally carried out by reacting a purine nucleotide with an alkylating agent. When the alkylation is carried out at the nucleotide level, the quaternized nitrogen purine nucleotide can be chemically or enzymatically incorporated into the polynucleotide sequence.

The alkylations should be carried out at a pH ranging from about 6.0 to about 8.0. At a pH below 6.0, the acidity will adversely affect the product. When alkylation is carried out at the nucleotide level, the acidity will promote cleavage of the quaternized nitrogen purine from the sugar. This cleavage will decrease the yield of product that can be utilized as a substrate by the polymerase enzyme (polymerase enzymes require a substrate comprising a base, sugar, and phosphate group for enzymatic incorporation). When alkylation is carried out at the polynucleotide level, the acidity will promote cross-linking of the bases to the sugar distorting the polynucleotide tertiary structure, and precluding the use of the polynucleotide as a probe. If the alkylation is done at a pH greater than about 8.0, the polynucleotide sequence can be cut at the site of the alkylated base, generating small ineffective pieces of probe. The polynucleotide sequence comprising a quaternized nitrogen purine nucleotide is referred to herein as a alkylated polynucleotide sequence.

Alkylating agents that are suitable for use with this invention include, for example, alkyl halides, dialkyl sulfates, dialkylsulfonates, and diazomethane. Preferred alkylating agents are the alkyl iodides, alkyl halides, dialkyl sulfates and diazomethane. Particularly preferred are methyl iodide, dimethyl sulfate, and diazomethane.

The purine nitrogen quaternized when alkylation is carried out at the nucleotide level should be the N-7. This is because alkylation at N-1 or at N-3 will distort the template and interfere with the base pairing of the purine to its complementary pyrimidine base. This base pairing is a requirement for incorporation by nick translation. The purine nitrogen that is quaternized when alkylation is carried out at the polynucleotide level can be N-1, N-3, or N-7 because the purine is already incorporated into the polynucleotide.

It is preferred to purify the alkylated nucleotide prior to incorporation into the polynucleotide sequence, and to purify the alkylated polynucleotide prior to depurination. The alkylated nucleotide can be purified by chromatography, for example, ion exchange chromatography, and Sephadex chromatography. The alkylated polynucleotide can be purified by, for example, dialysis, ethanol precipitation, and gel chromatography. Methods for carrying out the alkylation and purification steps are well known in the art.

Alkylated nucleotides, following purification, can be enzymatically incorporated into a polynucleotide sequence by, for example, the method of nick translation with polymerase enzymes. This method is well known in the art. See Rigby, P.W.J., Dieckmann, M., Rhodes, C., and Berg, P., Labeling Deoxyribonucleic Acid To High Specific Activity in vitro By Nick Translation With DNA Polymerase I, J. Mol.Biol. 113, pp 237-251 (1977).

The purified quaternized polynucleotide sequence (the polynucleotide sequence containing at least one quaternized purine nitrogen) is then selectively depurinated to cleave only the alkylated purines and to form sugar aldehydes. Selective depurination is carried out by heating the polynucleotide sequence in a solution whose pH is from about 5.0 to about 7.5, preferably at a pH from about 6.0 to about 7.5, and at a temperature from about 35°C to about 60°C, preferably from about 40°C to about 60°C. Following depurination, the polynucleotide sequence is purified by ethanol precipitation.

The selective depurination rate depends on the temperature. For example, at 37°C and pH 7.4, the half-lives for 7-methyldeoxyguanosine and 3-methyldeoxyadenosine for depurination from DNA are 69 hours and 15 hours, respectively. See Methylation of Deoxyribonucleic Acid by Diazomethane by E. Kriep and P. Emmelot, Biochemica and Biophysica Acta, 91, pp 59-66 (1964) which is hereby incorporated by reference. At 85°C after 15 minutes, 7-methyldeoxyguanosine is completely cleaved to 7-methylguanine and deoxyribose. For this invention, the extent of depurination at a given temperature can be readily determined as described in the above paper.

Another method for generating sugar aldehydes comprises cleaving uracil from polynucleotides. Uracil is a natural component of polyribonucleotides (RNA). The uracil can be incorporated into polydeoxyribonucleotides (DNA) by nick translating the polynucleotide sequence in the presence of deoxyuridylic acid triphosphate. This deoxyribonucleotide can be incorporated in lieu of thymidylic acid triphosphate. The amount of incorporated deoxyuridylic acid varies with the ratio of the deoxyuridylic acid/thymidylic acid in the reaction mixture.

The uracil is degraded with hydroxylamine to generate a sugar oxime at the 1-position of the sugar. Thus, RNA yields ribosyloxime moiety at the site of cleavage, while DNA yields a deoxyribosyloxime moiety at the site of cleavage. It is preferred that the hydroxylamine concentration be about 10M. The temperature can range from about 0°C to about 40°C, with about 5°C to about 15°C being preferred. Particularly preferred is about 10°C. The pH can range from about 9.0 to about 11.0, with about pH 10.0 being preferred. For further details about hydroxylamine cleavage, see the articles, "The Chemical Modification of Nucleic Acids; I. The Preparation of Deuridylic RNA's," N.K. Kochetkor, E.I. Budowsky, V.P. Demushkin, M.F. Turchinsky, N.A. Simukova, and E.D. Sverdlor, Biochem. Biophys. Acta, 142, 35-46 (1967) and, "The Extent and Specificity of the Degradation of Polynucleotide Chain Under the Conditions of the Preparation of Deuridylic RNA," E.I. Budowsky, N.A. Simukova, and L.I. Guskova, Biochem, Biophys. Acta, 166, 755-756 (1968).

Following degradation of the uracil, the excess hydroxylamine is removed by, for example, dialysis or chromatography. This is to prevent unreacted hydroxylamine from later interfering with the exchange reaction, as discussed hereinbelow.

The polynucleotide sequence from which a uracil base has been cleaved, contains a sugar oxime that must be converted to a sugar aldehyde. This is achieved by mixing the polynucleotide sequence with an excess of a ketone-containing compound, for example, acetone, or cyclohexanone. The concentration of the ketone-containing compound should be between about 0.1M to about 0.5M and the pH of the solution should be from about 4.5 to about 5.0, preferably about 5.0. Acetate buffer is suitable for this pH. An exchange occurs in which the oxime is transferred to the added ketone-containing compound, simultaneously generating a sugar aldehyde in the polynucleotide sequence. After the mixture has been allowed to stand at room temperature for about two hours, the ketone-containing compound is distilled off under vacuum.

The signalling moiety or bridging moiety must be present in the reaction mixture when the alkylated purine base is cleaved to generate the sugar aldehyde. This permits attachment of the signalling moiety to occur simultaneously with generation of the sugar aldehyde and limits the possibility that some cross-linking between a base and the sugar will occur. The signalling moiety must be added after cleavage of the uracil bases.

When uracil is the base removed, attachment of the signalling moiety to the polynucleotide sequence is carried out by dissolving the polynucleotide in a dilute salt solution, for example, 0.01M sodium chloride. Signalling moiety at a concentration greater than about 40mM is then added. The signalling moiety contains a functional group that can react with the sugar aldehyde when no bridging moiety is to be used. Suitable groups include $NH_2$-NH-, $NH_2$-NH-$CH_2$-and HS- , $NH_2$-NH-$CH_2$-being preferred. The pH is adjusted to be from about 4.5 to about 5.0, preferably to about 5.0. After the solution has been allowed to stand at room temperature for about 1 hour, the pH of the solution is brought up to about 7.0, and the polynucleotide sequence is dialyzed against saline sodium citrate buffer (1 SSC). The polynucleotide sequence is then precipitated with ethanol and collected.

It is advantageous to attach a signalling moiety to a sugar of a polynucleotide sequence because the sugar is at the exterior of the helix. Thus, there is no steric interference between the signalling moiety and the paired bases. Furthermore, it is believed that because the extent of methylated purine or uracil incorporated into a polynucleotide probe can be controlled by enzymatic incorporation, ratios of signalling moiety to base as high as 1:20 can be achieved without interference with subsequent polynucleotide hybridization.

The "signalling moiety" can encompass virtually any of the signal generating systems used in the prior art, and any system to be developed in the future. It comprises a moiety which generates a signal itself (for example, a radiolabel), or a moiety which upon further reaction or manipulation will give rise to a signal (for example, an enzyme-linked system). The signalling moiety must, however, have a functional group by means of which it can be attached to the sugar or bridging moiety.

The signalling moiety may thus comprise a radiolabel (e.g., $^{14}$C, $^{32}$P, $^3$H, and the like), an enzyme (e.g., peroxidase, alkaline or acid phosphatase, and the like), a bacterial label, a fluorescent label, an antibody (which may be used in a double antibody system), an antigen (to be used with a labeled antibody), a small molecule such as biotin (to be used with an avidin, streptavidin, or antibiotin system), a latex particle (to be used in a buoyancy or latex agglutination system), an electron dense compound such as ferritin (to be used with electron microscopy), or any combinations or permutations thereof.

Means for attaching signalling moieties to a sugar are illustrated in Example II. Other methods are known in the art. See, for example, the article by Levinson, cited hereinabove.

The following examples are by way of illustration and not by way of limitation.

### EXAMPLE I:

### Synthesis of 7-methyldeoxyguanosine containing DNA

Thirteen micromoles of deoxyguanosine triphosphate (dGTP) were dissolved in 2 ml of a 0.5M sodium cacodylate solution containing 100 microliters of dimethylsulfate and allowed to remain at room temperature. HPLC analysis showed that 80% of the dGTP was methylated. The mixture was diluted with water to 8 ml total and loaded onto a diethylaminoethyl (DEAE) cellulose column in the chloride form (3.5 ml). The column was washed with 10 ml of 0.05 M potassium chloride (KCl). Approximately 70% of the material (based on the absorption at 260 nm) did not bind to the matrix. This material was reloaded onto another DEAE cellulose column (3.5 ml) and washed with 20 ml of 0.02M KCl. The methylated dGTP was then eluted off with 0.3M KCl. The yield was about 75% of the total methylated dGTP.

The methylated dGTP was incorporated into DNA by nick translation. The following vials were prepared.

(1) DNase I:

0.25 $\mu$g/ml in buffer of vial #2.

(2) DNase I Dilution Buffer:

10mM Tris HCl pH 7.5, 1 mg/ml nuclease-free BSA.

(3) $^3$H-dATP (15-25 Ci/mmol):

deoxy (8-$^3$H) adenosine 5'-triphosphate, ammonium salt in 50% aqueous ethanol, 0.25 $\mu$Ci/$\mu$l.

(4) 10X Nick Translation Buffer:

0.5M Tris-HCl pH 7.5, 50mM MgCl$_2$

(5) Deoxynucleotide Solution:

0. 3mM dATP, 0.3mM dTTP, 0.3mM dCTP in 50mM Tris-HCl pH 7.5.

(6) 0.3mM methylated dGTP in 50mM Tris, pH 7.5.

(7) 0.3mM dGTP in 50mM Tris, pH 7.5.

(8) 0.3mM methylated dGTP and 0.27mM dGTP in 50mM Tris pH 7.5.

(9) DNA Polymerase I:

3 units/$\mu$l in 0.1M sodium phosphate buffer pH 7.2, 50% glycerol (v/v), 1.0mM dithiothreitol, DTT.

(10) Nick Translation Stop Buffer:

0.2M EDTA

(11) Control DNA:

0.25 mg/ml lambda DNA in 50mM Tris-HCl pH 7.4.

The reaction and controls were carried out in 6 tubes. Tube 1 contained the 7 following additions:

A.    A $^3$H-dATP solution prepared by lyophilizing 12 $\mu$ls of vial #3 and adding 50 $\mu$ls of water.

B.    5 $\mu$ls of a 10-fold dilution of vial #4.

C.    5 $\mu$ls of vial #5.

D.    5 $\mu$ls of vial #7.

E.    4 $\mu$ls of vial #11.

F.    4 $\mu$ls of vial #9.

G.    4 $\mu$ls from a solution containing 2 $\mu$ls of vial #1 diluted with 38 $\mu$ls of vial #2.

Tube 2 was prepared differently from tube 1 in that addition E was omitted.

Tube 3 did not have additions D or E.

Tube 4 did not have addition D.

Tube 5 contained 5 $\mu$ls of vial #6 instead of 5 $\mu$ls of vial #7 for addition D.

Tube 6 contained 5 $\mu$ls of vial 8 for addition D.

The tubes were incubated at 14ºC for 2 hours and stopped by adding 5$\mu$l of vial 10. The enzymes were then inactivated by incubation at 65ºC for 10 minutes.

The percent of nucleotide incorporated was determined as follows:

A 2 microliter aliquot was placed into a 5 ml plastic tube to which 10 micrograms of sonicated calf thymus DNA had been added. 1 ml cold 5% (w/v) trichloroacetic acid (TCA), and 25mM sodium pyrophosphate were added. The tube was kept on ice for 15 minutes. The solution was filtered through glass fiber filters. The filters were washed thoroughly with 2% TCA, 10mM sodium pyrophosphate, and thoroughly dried. Toluene-based liquid scintillation cocktail was added to cover the filter, and the filter was counted in a liquid scintillation counter. The total radioactivity in the reaction mixture was determined by transfering a second 2 microliter aliquot into 150 microliters of water, spotting the entire 152 microliters directly (without filtration) on a glass fiber filter, drying and counting the filter.

Using the following equation, the percent incorporated can be determined:

Percent Incorporation

$$= \frac{(ppt\ cpm)\ x\ (330)}{(specific\ x\ (ng\ DNA\ x\ (mol \qquad (total\ cpm}\ x\ 100$$

activity      per      fraction  input dpm)

³H–dATP)   aliquot)   T)

| | |
|---|---|
| ppt cpm = | cpm per 2 $\mu$l aliquot after TCA precipitation, |
| 330 = | average molecular weight of nucleotide, |
| sp. act $^3$H-dATP = | in dpm per nmole ($l\mu$ci $= 2.2 \times 10^6$ dpm). Note: Specific activity per reaction is diluted by cold dATP and must be corrected for this dilution from the original specific activity of the $^3$H-dATP For the $^3$H-dATP used, the calculation is as follows: |

$$\frac{3\mu Ci\ x\ 2.26\ x\ 10^6\ dpm/\mu Ci}{x\ 10^6\ dpm/nmol\ 1.65\ nmol\ total\ dATP} = 4.4$$

mol

| | |
|---|---|
| Fraction T = | assume 0.25 if mol fraction T of your probe is unknown, |
| Total cpm = | cpm of aliquot not TCA precipitated, |
| Input dpm = | per 2 $\mu$l aliquot, and |

$$\underline{\frac{Total\ cpm}{Input\ dpm}} = efficiency\ of\ counting.$$

| | |
|---|---|
| ppt cpm = | counts per minute per 2 $\mu$l aliquot after TCA precipitation |
| dpm = | disintegration per minute |
| ppt = | parts per thousand |
| tca = | trichloracetic acid, |

Desirable levels of incorporation are in the range of 20-50%. The results obtained for tubes 1-6 were 0.3%, 7.8%, 5.6%, 59.9%, 19.8%, and 63.6% respectively.

To ascertain that methyl G, and not an unreacted subset of that reagent was being incorporated, 2-20 $\mu$l aliquots were removed from A and B, after these DNAs had been purified on Sephadex G50. 100 $\mu$l of 0.1M piperidine was added to each sample. One of each set was then heated for 60 mintes at 90°C, while the other sample was kept on ice. The rationale for this is as follows:

The Maxam-Gilbert sequencing chemistry uses DMSO to methylate GMP (partially). Heating in piperidine results in cleavage of the strand, resulting in a "ladder" on auto-radiography. In our case, if the methylated G's are randomly distributed along the DNA, the piperidine would cleave the DNA at the methylated sites resulting in the generation of small fragments. Sufficiently small fragments would not be TCA precipitable and would not yield counts. The unheated counterpart which is large, would be TCA-precipitable and would serve as a control.

Both the heated and non-heated samples were suspended in 10% TCA with 100 $\mu$g/ml carrier DNA added. After 30 minutes on ice the filters were dried and counted. The results are shown below:

| | |
|---|---|
| A. Heated DNA | 2244 cpm |
| A. Unheated DNA | 5888 cpm |
| B. Heated DNA | 3624 cpm |
| B. Unheated DNA | 9870 cpm |

The results clearly indicate that the heated samples were cleaved by the piperidine. Therefore, 7-methyl GMP had been incorporated into the DNA.

**EXAMPLE II**:

**Synthesis of an allylgalactoside-acrylamide copolymer bound to lucifer yellow**

Fifty grams of galactose was mixed with 100 milliliters of allyl alcohol containing 3% (w/v) HCl, and heated for 3 1/2 hours while stirring at 80ºC. The HCl was neutralized with concentrated ammonia and the allyl alcohol distilled off under reduced pressure (0.5 mm Hg), at 75ºC. The remaining syrup was mixed with 20 milliliters of $H_2O$ and heated at 75ºC until dissolved.

The solution was mixed with 80 grams of cellulose and stirred until homogeneous. The mixture was then stirred three times for ten minute intervals with 300 ml of dry acetone. The acetone extracts were combined and the acetone distilled off leaving a brown syrup. Fifteen ml of ethanol were added and the mixture was heated until the syrup dissolved. The volume was reduced to 50 ml under reduced pressure. The solution was cooled to 4ºC and seeded with a few crystals of alpha-methylmannoside. Crystallization of the allylgalactoside was complete after two days. The yield was 9.2 g (15 percent).

200 milligrams of acrylamide, 100 milligrams of allylgalactoside, and 50 microliters of ammonium persulfate (100 milligrams/ml) were mixed in 2 ml of total volume. The solution was heated for 20 minutes at 65°C. The viscosity of the solution increased with reaction time. The solution was cooled and the residue dialyzed against 2 liters of $H_2O$ for two days. The undialyzable material comprised the copolymer.

A tube containing 4 mg of the copolymer and 4.8 units of galactose oxidase (calcium and magnesium free) in phosphate buffered saline (PBS) was incubated for 30 minutes at 37ºC. A one hundred $\mu$l aliquot was removed from the tube and diluted with 500 $\mu$l of PBS. Four $\mu$l of a 27.3 micromolar lucifer yellow solution were added to the tube, and the test tube was allowed to stand for 18 hours. The solution was chromatographed through a G50 column. The peak containing the adduct eluted with tailing due to the nonhomogeneity of the copolymer. The tailing portion was not collected. An aliquot of 1.4 ml was removed from the adduct peak and the UV absorbance measured at 435 nm. The O.D. was 0.405 which corresponds to 0.042 millimoles of dye since the molar extinction coefficient of lucifer yellow dye at 435 nm is 13,500. Experiments carried out with sugar aldehydes and lucifer yellow showed that a molecule of dye was bound to every aldehyde molecule. Since in the 100 $\mu$l aliquot there was 9.2 milligrams of galactose to 660 mgs of acrylamide, there were 22.6 molecules of acrylamide to every 1 molecule of galactose.

Albeit the preferred embodiment of the present invention has been described above, it is contemplated that other alterations and modifications may become apparent to those skilled in the art after having read the above disclosure. For example, other reagents that can cleave pyrimidines may be found, or other means may be discovered for quaternizing a purine nitrogen so as to facilitate depurination. It is therefore intended that the appended claims be interpreted as covering all such alterations and modifications as fall within the true spirit and scope of this invention.

**Claims**

1. A method of labeling an alkylated polynucleotide sequence comprising generating a sugar aldehyde by cleaving a quaternized nitrogen purine nucleotide from said sequence at a pH from about 5.0 to about 7.5 and at a temperature from about 35ºC to about 60ºC in the presence of a signalling moiety whereby said signalling moiety becomes attached to said sugar aldehyde.

2. The method of Claim 1 wherein said quaternized nitrogen purine nucleotide in said modified sequence is obtained by contacting a polynucleotide sequence with an alkylating agent or by incorporating a quaternized nitrogen purine nucleotide into a polynucleotide sequence.

3. The method of Claim 2 wherein said quaternized nitrogen purine nucleotide is formed by contacting a purine nucleotide with an alkylating agent.

4. The method of Claim 2 or 3 wherein said alkylating agent is selected from the group consisting of diazomethane, dialkyl sulfates, dialkyl sulfonates, and alkyl halides, preferably, diazomethane, dimethyl sulfate, and methyl iodide.

5. The method of Claim 3 or 4 wherein said quaternized nitrogen purine nucleotide is 7-methylguanylic acid or 7-methyladenylic acid and is incorporated into said polynucleotide sequence by means of nick translation.

6. A method of labeling a polynucleotide sequence comprising the steps of:
(a) cleaving at least one uracil base from said polynucleotide sequence with hydroxylamine to generate a sugar oxime at the 1-position of said sugar;
(b) contacting said polynucleotide sequence with a ketone-containing compound to covert said sugar oxime to a sugar aldehyde; and
(c) attaching a signalling moiety to said sugar aldehyde.

7. The method of Claim 6 wherein said polynucleotide sequence is DNA and said uracil base is incorporated as deoxyuridylic acid.

8. The method of Claim 6 or 7 wherein said cleaving step is carried out in a 10M hydroxylamine solution, wherein the temperature of said solution is from about 0ºC to about 40ºC, preferably from about 5ºC to about 15ºC, and more preferably about 10ºC and the pH of said solution is from about 9.0 to about 11.0.

9. The method of any one of claims 6 to 8 wherein said contacting step is carried out in a solution wherein the concentration of said ketone-containing compound is from about 0.1M to about 0.5M.

10. The method of any one of the preceding claims wherein said signalling moiety comprises a functional group selected from the group consisting of $NH_2$-NH-, $NH_2$-NH-$CH_2$-and HS-.

**Revendications**

1. Une méthode de marquage d'une séquence polynucléotidique alkylée comprenant de générer un aldéhyde de sucre en clivant un nucléotide purique dont l'azote est quaternaire de ladite séquence à un pH d'environ 5,0 à 7,5 et à une température d'environ 35°C à environ 60°C en présence d'une unité de signalisation par laquelle ladite unité de signalisation se retrouve attachée au dit aldéhyde de sucre.

2. La méthode de la revendication 1 dans laquelle ledit nucléotide purique à azote quaternaire dans ladite séquence modifiée est obtenu en mettant en contact une séquence de polynucléotides avec un agent d'alkylation ou en incorporant un nucléotide purique à azote quaternaire dans une séquence de polynucléotides.

3. La méthode de la revendication 2 dans laquelle ledit nucléotide purique à azote quaternaire est formé en mettant en contact un nucléotide purique avec un agent d'alkylation.

4. La méthode de la revendication 2 ou 3 dans laquelle ledit agent d'alkylation est choisi dans le groupe consistant en le diazométhane, les sulfates de dialkyle,les sulfonates de dialkyle, et les halogénures d'alkyle, de préférence le diazométhane, le sulfate de diméthyle, et l'iodure de méthyle.

5. La méthode de la revendication 3 ou 4 dans laquelle ledit nucléotide purique à azote quaternaire est l'acide 7-méthylguanylique ou l'acide 7-méthyladénylique et est incorporé dans ladite séquence de polynucléotides au moyen d'une "nick translation".

6. Une méthode de marquage d'une séquence de polynucléotides comprenant les étapes de :
(a) cliver au moins une base uracile de ladite séquence de polynucléotides avec de l'hydroxylamine pour générer un oxime de sucre en position 1 dudit sucre ;
(b) mettre en contact ladite séquence de polynucléotides avec un composé contenant une cétone pour convertir ledit oxime de sucre en un aldéhyde de sucre ; et
(c) attacher une unité de signalisation au dit aldéhyde de sucre.

7. La méthode de la revendication 6 dans laquelle ladite séquence polynucléotidique est de l'ADN et ladite base uracile est incorporée sous forme d'acide déoxyuridylique.

8. La méthode de la revendication 6 ou 7 dans laquelle ladite étape de clivage est effectuée dans une solution d'hydroxylamine 10M, dans laquelle la température de ladite solution va d'environ 0°C à environ 40°C, de préférence d'environ 5°C à environ 15°C, et de manière plus préférable autour de 10°C et le pH de ladite solution va d'environ 9,0 à environ 11,0.

9. La-méthode de n'importe laquelle des revendications 6 à 8 dans laquelle ladite étape de mise en contact est pratiquée dans une solution dans laquelle la concentration dudit composé contenant une cétone va d'environ 0,1M à environ 0,5M.

10. La méthode de n'importe laquelle des revendications précédentes dans laquelle ladite unité de signalisation comprend un groupemement fonctionnel choisi dans le groupe consistant en $NH_2-NH-$, $NH_2-NH-CH_2-$ et HS-.

**Patentansprüche**

1. Verfahren zur Markierung einer alkylierten Polynukleotidsequenz, umfassend die Bildung eines Zuckeraldehyds durch Abspaltung eines quaternisierten Stickstoff-Purinnukleotids von dieser Sequenz bei einem pH-Wert von etwa 5,0 bis etwa 7,5 und einer Temperatur von etwa 35°C bis etwa 60°C in Gegenwart einer Signaleinheit, wobei die Signaleinheit mit dem Zuckeraldehyd verknüpft wird.

2. Verfahren nach Anspruch 1, wobei das quaternisierte Stickstoff-Purinnukleotid in der modifizierten Sequenz durch Inkontaktbringen einer Polynukleotidsequenz mit einem Alkylierungsmittel oder durch Einbau eines quaternisierten Stickstoff-Purinnukleotids in eine Polynukleotidsequenz erhalten wird.

3. Verfahren nach Anspruch 2, wobei das quaternisierte Stickstoff-Purinnukleotid durch Inkontaktbringen eines Purinnukleotids mit einem Alkylierungsmittel gebildet wird.

4. Verfahren nach Anspruch 2 oder 3, wobei das Alkylierungsmittel aus der Gruppe Diazomethan, Dialkylsulfate, Dialkylsulfonate und Alkylhalogenide, vorzugsweise Diazomethan, Dimethylsulfat und Methyljodid ausgewählt ist.

5. Verfahren nach Anspruch 3 oder 4, wobei das quaternisierte Stickstoff-Purinnukleotid 7-Methylguanylsäure oder 7-Methyladenylsäure ist und in die Polynukleotidsequenz mittels Nick-Translation eingebaut wird.

6. Verfahren zur Markierung einer Polynukleotidsequenz, umfassend die folgenden Schritte:
   (a) Abspaltung mindestens einer Uracil-Base aus der Polynukleotidsequenz mit Hydroxylamin, um ein Zuckeroxim in der 1-Stellung des Zuckers zu erhalten;
   (b) Inkontaktbringen der Polynukleotidsequenz mit einer Keton enthaltenden Verbindung, um das Zuckeroxim in einen Zuckeraldehyd umzuwandeln; und
   (c) Anknüpfung einer Signaleinheit an den Zuckeraldehyd.

7. Verfahren nach Anspruch 6, wobei die Polynukleotidsequenz DNA ist und die Uracilbase als Desoxyuridylsäure eingebaut wird.

8. Verfahren nach Anspruch 6 oder 7, wobei der Abspaltungsschritt in einer 10 M Hydroxylaminlösung durchgeführt wird, wobei die Temperatur der Lösung etwa 0°C bis etwa 40°C, vorzugsweise etwa 5°C bis etwa 15°C, besonders bevorzugt etwa 10°C beträgt, und der pH-Wert der Lösung etwa 9,0 bis etwa 11,0 beträgt.

9. Verfahren nach einem der Ansprüche 6 bis 8, wobei der Schritt des Inkontaktbringens in eine Lösung durchgeführt wird, in der die Konzentration der Keton enthaltenden Verbindung etwa 0,1 M bis etwa 0,5 M beträgt.

10. Verfahren nach einem der vorangehenden Ansprüche, wobei die Signaleinheit eine funktionelle Gruppe ist, die ausgewählt ist aus $NH_2-NH-$, $NH_2-NH-CH_2-$ und HS-.